# EUROPÄISCHE PATENTSCHRIFT

(11) **EP 3 569 181 B1**
(45) Veröffentlichungstag und Bekanntmachung des Hinweises auf die Patenterteilung: **07.02.2024**
(21) Anmeldenummer: 18173362.7
(22) Anmeldetag: 18.05.2018
(51) Int. Cl.: A61B 50/36, A61B 17/3217, A61M 5/32, B65F 1/14

(54) **VERFAHREN ZUM RECYCLEN VON METALLISCHEN MEDIZINISCHEN GERÄTEN**
METHOD FOR RECYCLING METALLIC MEDICAL DEVICES
PROCÉDÉ DE RECYCLAGE D'APPAREILS MÉDICAUX MÉTALLIQUES

(43) Veröffentlichungstag der Anmeldung: 20.11.2019
(73) Patentinhaber: Scholz Medical GmbH, 83624 Otterfing (DE)
(72) Erfinder: LORKE, Werner W., 60486 Frankfurt (DE)
(74) Vertreter: Kramer Barske Schmidtchen Patentanwälte PartG mbB

(56) Entgegenhaltungen:
- EP-A1- 3 320 991
- DE-A1-102015 016 182
- DE-A1-102016 005 649
- DE-U1-202017 103 735
- Anonymous: "Einmal-Instrumente - Exklusiv mit dem Scholz Recycling-System SReS > SMS medipool (full page)", , 20. März 2018 (2018-03-20), XP055517854, Gefunden im Internet: URL:https://sms-medipool.de/einmal-instrum ente-exklusiv-mit-sres-scholz-recycling-sy stem/ [gefunden am 2018-10-22] -& Anonymous: "SReS Scholz Recycling System für chirurgische Einmal-Instrumente", , 20. März 2018 (2018-03-20), XP055517841, Gefunden im Internet: URL:https://sms-medipool.de/wp-content/upl oads/2018/03/medimex_ProspektSReS_ScholzRe cyclingSystem_web_30_01_2018.pdf [gefunden am 2018-10-22] -& Anonymous: "Sterile Einmal-Instrumente", , 20. März 2018 (2018-03-20), XP055517845, Gefunden im Internet: URL:https://sms-medipool.de/wp-content/upl oads/2018/03/SMS-Broschuere-Einmal-Instrum ente-V1-WEB.pdf [gefunden am 2018-10-22]
- Anonymous: "medimex: NEU im Sortiment: medimex Einmal-Instrumente", , 13. Juli 2017 (2017-07-13), XP055517827, Gefunden im Internet: URL:https://medimex.de/index.php?id=79&L=1 %252522&tx_ttnews%5Btt_news%5D=52&cHash=c9 b0e7f2cd1ee30fbbc9e3b72233a084 [gefunden am 2018-10-22] -& Anonymous: "medimex Einmal-Instrumente", , 13. Juli 2017 (2017-07-13), XP055517830, Gefunden im Internet: URL:https://medimex.de/fileadmin/inhalte/D ownloads_neu/medimex-Einmalinstrumente_130 72017_web.pdf [gefunden am 2018-10-22]

## Beschreibung

Die vorliegende Erfindung betrifft ein Verfahren zur, insbesondere Ressourcen schonenden bzw. Wertstoff erhaltenden, recycelnden Entsorgung, von, insbesondere nicht-infektiös kontaminierten, metallischen medizinischen Geräten aus insbesondere Kliniken, medizinischen Ambulanzen und/oder Arztpraxen.

Metallische Klinikabfälle wie medizinische Implantate, Endoprothesen und chirurgischen Ein- oder Mehrweg-Instrumente werden im Allgemeinen je nach Kontaminationsgrad einer hierfür vorgesehenen Endlagerstätte zugeführt. Nicht selten werden solche metallischen Klinikabfälle zunächst zusammen mit weiteren Klinikabfällen einer Müllverbrennung unterzogen, und die hierbei erhaltenen Rückstände werden sodann deponiert. Auch geht man gegenwärtig davon aus, dass pro Jahr in Deutschland etwa 50.000 kg medizinische Implantate und defekte chirurgische Mehrweg-Instrumente unwiederbringlich im Müll landen. Darüber hinaus nimmt der Verbrauch von chirurgischen Einweginstrumenten - vorwiegend aus chromhaltigen Edelstählen gefertigt - insbesondere in Kliniken, Ambulanzen und Arztpraxen seit Jahren zu. Die Menge an Edelstahl, die allein in Deutschland in 2014 für chirurgische Einweginstrumente verwendet wurde, lag bei etwa 8000 t. Auch dieser Edelstahlabfall wird regelmäßig über den allgemeinen Klinikabfall in Müllverbrennungsanlagen entsorgt. Die metallischen Klinikabfälle werden folglich dem Wertstoffkreislauf dauerhaft entzogen.

Ein weiteres Verfahren zum Recyclen von metallischen medizinischen Geräten ist bekannt aus der EP 3 320 991 A1. Weiterhin offenbaren die DE 10 2015 016182 A1, DE 10 2016 005649 A1 und DE 20 2017 103735 U1 Vorrichtungen und Verfahren in Bezug auf Verfahren zum Recyclen von metallischen medizinischen Geräten.

Der vorliegenden Erfindung lag die Aufgabe zugrunde, die aus dem Stand der Technik bekannten Nachteile abzustellen oder zu mindern. Insbesondere lag der Erfindung die Aufgabe zugrunde, die Handhabung von metallischen Klinikabfällen zu verbessern.

Diese Aufgabe wird gelöst mit einem Verfahren nach Anspruch 1. Weiterbildungen sind in den abhängigen Ansprüchen angegeben.

Mit dem beanspruchten Verfahren ist es möglich, dass die abschnittsweise bzw. vorzugsweise im Wesentlichen vollständig flexiblen Sammelbehältnisse (A) ebenfalls dem Aufschmelzprozess der darin gesammelten Metalle der benutzten und/kontaminierten metallischen medizinischen Geräte unterworfen werden, d.h. diese benutzten und/kontaminierten metallischen medizinischen Geräte werden den Sammelbehältnissen (A) nicht entnommen, sondern in diesen Sammelbehältnissen (A) vorliegend dem Schritt g) unterworfen. Auch ist es möglich, , dass die abschnittsweise oder vorzugsweise im Wesentlichen vollständig flexiblen Einweggebinde (B), sofern eingesetzt, zusammen mit den darin befindlichen Sammelbehältnissen (A) enthaltend die benutzten/oder kontaminierten metallischen medizinischen Geräte dem Schritt (g) unterworfen werden. In gleicher Weise ist es möglich, die mit den Sammelbehältnissen (A) befüllten Kunststoff-/oder Metall-Einweggebinde (B), ohne diese zuvor zu entleeren, dem Schritt g) zu unterwerfen. Optional ist dieses auch mit den starren Kunststoff- und insbesondere den Metall-Mehrweggebinden (B) möglich, insbesondere dann, wenn diese Gebinde alters- und/oder abnutzungsbedingt nicht mehr für die erneute Verwendung als Aufnahme- und Transportbehältnis geeignet sind. In allen anderen Fällen hat es sich als zweckmäßig erwiesen, die starren Kunststoff- und/oder Metall-Mehrweggebinde (B) für das Sammeln und den Transport von mit benutzten und/oder kontaminierten metallischen medizinischen Geräten befüllten Sammelbehältnissen (A) wiederzuverwenden.

Für viele Anwendungen des erfindungsgemäßen Verfahrens hat es sich als zweckmäßig erwiesen, das abschnittsweise oder vorzugsweise im Wesentlichen vollständig flexible Einweggebinde (B) in einem starren Aufnahmebehältnis, insbesondere einer Metallgitterbox, vorzusehen. Auf diese Weise lässt sich das Sammeln und der Transport der mit benutzten und/oder kontaminierten metallischen medizinischen Geräten befüllten Sammelbehältnisse (A) in diesem Einweggebinde (B) besonders sicher und unproblematisch bewerkstelligen. Hierbei kann in einer weiteren Ausführungsform das abschnittsweise oder vorzugsweise im Wesentlichen vollständig flexible Einweggebinde oder das starre Aufnahmebehältnis auf einer Tragevorrichtung vorliegen, beispielsweise auf einer Palette. Hierdurch lässt sich der Transport vereinfachen.

In einer geeigneten Ausgestaltung des erfindungsgemäßen Verfahrens umfasst dieses ferner den Schritt der Herstellung von metallischen medizinischen Geräten und/oder von metallischen Gebinden aus den in Schritt g) rückgewonnenen Metallen. Dieser Schritt kann dabei beispielsweise auch die Bereitstellung von aus in Schritt g) rückgewonnenen Metallen bzw. Metalllegierungen, und damit von in ihrer chemischen Zusammensetzung bekannten Metall- bzw. Legierungszuschlägen für u.a. die Herstellung von metallischen medizinischen Geräten und/oder von metallischen Gebinden umfassen.

Das Sammelbehältnis (A) ist in einer bevorzugten Ausgestaltung nicht nur abschnittsweise, sondern im Wesentlichen vollständig aus flexiblen, beispielsweise knick-, dehn- und/oder faltbaren, Wandungsmaterialien gebildet bzw. im Wesentlichen als Ganzes flexibel ausgestaltet.

Metallische medizinische Geräte, die mit dem erfindungsgemäßen Verfahren grundsätzlich einem Recyclingprozess zugeführt werden können, umfassen z.B. gebrauchte und/oder nicht verwendete Endoprothesen, chirurgische Instrumente, insbesondere Mehrweginstrumente, medizinische Einweginstrumente, Herzschrittmacher und/oder orthopädische Zubehörteile. Bei den nach dem erfindungsgemäßen Verfahren behandelten metallischen medizinischen Geräten handelt es sich vorzugsweise um metallische Einweg-Instrumente und/oder um metallische Mehrweg-Instrumente, insbesondere um chirurgische Einweg-Instrumente. Mit dem erfindungsgemäßen Verfahren können insbesondere auch solche metallischen medizinischen Geräte einer Wiederverwertung zugeführt werden, die Titan, Kobalt, Chrom und/oder Nickel enthalten. Derartige metallische medizinische Geräte sind dabei häufig aus mit Titan, Kobalt, Chrom und/oder Nickel legierten Edelstählen gebildet. Mit dem erfindungsgemäßen Verfahren lassen sich ganz besonders bevorzugt derartige metallischen medizinischen Geräte, insbesondere metallische Einweg-Instrumente und metallische chirurgische Mehrweg-Instrumente, einer Wiederverwertung zuführen, die Chrom-und/oder Nickel-legierte Edelstähle enthalten.

Gemäß dem erfindungsgemäßen Verfahren sind solche, vorzugsweise im Wesentlichen vollständig, flexiblen, insbesondere verschließbaren, Sammelbehältnisse (A) besonders geeignet, die im Wesentlichen feuchtigkeitsbeständig, dicht, insbesondere flüssigkeitsdicht, perforationsresistent, reißfest und/oder dauerhaft verschließbar sind. Diese Sammelbehältnisse (A) sind zweckmäßiger Weise eingerichtet und ausgelegt, um benutzte und/oder kontaminierte metallische medizinische Geräte mit einem Gesamtgewicht im Bereich von 0,5 bis 5,0 kg, insbesondere im Bereich von 1,0 bis 3,0 kg, aufzunehmen.

In einer vorteilhaften Ausgestaltung umfassen geeignete Sammelbehältnisse (A) eine flexible Behältnisseitenwandung, Behältnisboden und einen dem Behältnisboden gegenüberliegenden verschlossenen Behältnisrand, wobei eine Behältnisöffnung näher beabstandet zum verschlossenen Behältnisrand als zum Behältnisboden in einem Abschnitt der Behältnisseitenwandung vorliegt. Dabei sind auch solche flexiblen Sammelbehältnisse (A) geeignet, die eine flexible Behältnisseitenwandung, einen flexiblen Behältnisboden, einen dem Behältnisboden gegenüberliegenden Behältnisrand und eine Behältnisöffnung enthalten. In zweckmäßigen Ausgestaltungen kann dabei vorgesehen sein, dass die flexiblen Wandungsmaterialien des mindestens abschnittsweise, vorzugsweise im Wesentlichen vollständig, flexiblen Sammelbehältnisses (A) ausgewählt sind aus der Gruppe bestehend aus Polyolefinlagen, insbesondere Polyethylenlagen; Polyesterlagen, insbesondere PET-Lagen; Polyolefin/Polyester-Lagen, insbesondere PE/PET-Lagen; Faservlieslagen, insbesondere umfassend oder gebildet aus Polyolefinfasern, insbesondere Polyethylenfasern, und/oder Polyesterfasern, insbesondere PET-Fasern, Laminatsystemen, enthaltend mindestens eine Papierlage, mindestens eine feuchtigkeitsbeständig beschichtete und/oder mit einer Metalllage beschichtete Papierlage und/oder mindestens eine Kunststofffolienlage, insbesondere enthaltend mindestens eine Papierlage und mindestens eine Kunststofffolienlage und/oder mindestens eine feuchtigkeitsbeständig beschichtete und/oder mit einer Metalllage beschichtete weitere Papierlage; ein- oder beidseitig in eine Kunststofflage eingebetteten Metallgeweben, Metallgewirken oder Metallvliesen; Lagen aus Polyvinylbutyrat, insbesondere recyceltem Polyvinylbutyrat, sowie deren beliebigen Mischungen. Hierbei können die genannten flexiblen Wandungsmaterialien Behältnisseitenwandungen, insbesondere in Form von Vorderwand, Rückwand und gegebenenfalls Seitenwand oder -wänden, und insbesondere auch einschließlich Behältnisboden und/oder Behältnisrand, darstellen. Darüber hinaus kann auch auf solche flexiblen Sammelbehältnisse (A) zurückgegriffen werden, enthaltend eine Vorderseitenwandung und eine Rückseitenwandung und gegebenenfalls gegenüberliegende Seitenwandungen, insbesondere Seitenfaltenwandungen, einen den oberen, verschlossenen Behältnisrand bildenden Falz, über den Vorderseitenwandung und Rückseitenwandung miteinander verbunden sind, und/oder einen den oberen, verschlossenen Behältnisrand bildenden Behältnisabschnitt, über den Vorderseitenwandung und Rückseitenwandung miteinander in Verbindung stehen und/oder ineinander übergehen, ferner eine Öffnung, insbesondere schlitzförmige Öffnung, im oberen Bereich der Vorderseitenwandung sowie einen Behälterboden, der mindestens zwei, insbesondere eine Vielzahl an leporelloartig gefalteten Segmenten aufweist, wobei dieser Behälterboden vorzugsweise einstückig mit der Vorderseitenwandung oder der Rückseitenwandung vorliegt und/oder das endständige Segment der leporelloartig gefalteten Segmente des Behälterbodens mit der Innenwandung der Vorderseitenwandung oder der Rückseitenwandung verbunden, insbesondere verklebt, ist.

Bei den geeigneten mindestens abschnittsweise, vorzugsweise im Wesentlichen vollständig, flexiblen Sammelbehältnissen (A) liegt vorzugsweise mindestens ein Klebestreifen in dem Abschnitt des Vorderseitenareals zwischen der Beutelöffnung und der Falz oder dem Behältnisabschnitt auf derjenigen Seite des Vorderseitenareals vor, die bei einem aus der Materialbahn gebildeten Sammelbehältnis die Außenseite bildet. Auf diese Weise kann durch einfaches Umfalten des oberen Beutelabschnitts über die Beutelöffnung letztere erschlossen werden. In einer weiteren Ausführungsform kann vorgesehen sein, dass die mindestens abschnittsweise, vorzugsweise im Wesentlichen vollständig, flexiblen Sammelbehältnisse (A) nicht verschlossen werden. Dies ist insbesondere dann möglich, wenn die Einfüllöffnung, insbesondere die unterhalb des oberen Behältnisrandes angebrachte schlitz- oder streifenförmige Einfüllöffnung eine Entnahme oder ein Herausrutschen oder -fallen der darin gesammelten benutzten und/oder kontaminierten medizinischen Geräte erschwert oder nicht zulässt.

Als besonders geeignet hat sich ein solches Sammelbehältnis (A) für metallische medizinische Geräte, insbesondere für metallische chirurgische Einweginstrumente, erwiesen, umfassend eine flexible Behältnisvorderwand, eine flexible Behältnisrückwand, gegebenenfalls eine erste flexible Behältnisseitenwand, umfassend gegebenenfalls mindestens eine Seitenfalte, gegebenenfalls eine der ersten Behältnisseitenwand gegenüberliegende zweite flexible Behältnisseitenwand, gegebenenfalls umfassend mindestens eine Seitenfalte, ein unteres Behältnisende, umfassend einen Behältnisboden, und einen dem Behältnisboden gegenüberliegendes verschlossenes oberes Behältnisende, wobei näher beabstandet zum verschlossenen oberen Behältnisende als zum Behältnisboden in einem Abschnitt der Behältnisvorderwand oder der Behältnisrückwand eine Behältnisöffnung vorliegt, die sich in Richtung von der ersten Behältnisseitenwand zu der zweiten Behältnisseitenwand erstreckt, wobei Behältnisvorderwand und Behältnisrückwand am unteren Behältnisende, insbesondere über eine Faltung, einstückig ineinander übergehen oder wobei Behältnisvorderwand und Behältnisrückwand am unteren Behältnisende in einem sich von der ersten Behältnisseitenwand bis zur zweiten Behältnisseitenwand erstreckenden überlappenden ersten Abschnitt, insbesondere irreversibel, miteinander verbunden vorliegen und wobei der überlappende verbundene erste Abschnitt vollständig oder teilweise umgeschlagen auf und, insbesondere irreversibel, verbunden mit der Behältnisvorderwand oder die Behältnisrückwand vorliegt und wobei Behältnisvorderwand und Behältnisrückwand am verschlossenen oberen Behältnisende in einem sich von der ersten Behältnisseitenwand bis zur zweiten Behältnisseitenwand erstreckenden überlappenden zweiten Abschnitt zumindest teilweise, insbesondere irreversibel, miteinander verbunden vorliegen und wobei der überlappende zumindest teilweise verbundene zweite Abschnitt vollständig oder teilweise umgeschlagen auf und, insbesondere irreversibel, verbunden mit der Behältnisvorderwand oder die Behältnisrückwand vorliegt oder wobei ein am oberen Behältnisende über die Behältnisrückwand hinausragender Abschnitt der Behältnisvorderwand unter Ausbildung des verschlossenen oberen Endes umgeschlagen auf und, insbesondere irreversibel, verbunden mit der Behältnisrückwand vorliegt oder wobei ein am oberen Behältnisende über die Behältnisvorderwand hinausragender Abschnitt der Behältnisrückwand unter Ausbildung des verschlossenen oberen Endes umgeschlagen auf und, insbesondere irreversibel, verbunden mit der Behältnisvorderwand vorliegt. Hierbei sind solche Sammelbehältnisse (A) besonders bevorzugt, die ferner mindestens ein Verschlusselement an oder auf der Behältniswand, welche die Behältnisöffnung enthält, diesseits der Behältnisöffnung in Bezug auf das obere Behältnisende, ausgelegt und eingerichtet, um die Behältnisöffnung, insbesondere irreversibel, zu verschließen, und mindestens eine Haltevorrichtung, insbesondere Griff, Schlaufe oder Aufnahmeöffnung oder -loch, ausgelegt und eingerichtet zum temporären Befestigen des Sammelbehältnisses, so dass bei gattungsgemäßem Gebrauch die Behältnisöffnung unterhalb des oberen Behältnisende vorliegt, umfassen.

Auch insbesondere mit den vorangehend geschilderten Sammelbehältnissen lassen sich metallische medizinische Geräte in Form von Einweg- und/oder Mehrweg-Instrumenten, insbesondere Einweg-Instrumenten, sammeln und (zwischen) lagern. In den Sammelbehältnissen können z.B. metallische Kanülen, metallische Skalpelle, metallische Pinzetten, metallische Scheren, mechanische metallische Klemmen und/oder metallische Zangen gesammelt werden.

Im Zusammenhang mit den vorangehend geschilderten Ausführungsformen geeigneter Sammelbehältnisse (A) soll im Sinne der Erfindung unter irreversibel verschlossen bzw. irreversibel verbunden in einer besonders zweckmäßigen Ausgestaltung verstanden werden, dass ein Lösen der irreversibel verschlossenen oder irreversibel verbundenen Gegenstände nicht zerstörungsfrei möglich ist.

Besonders geeignete Sammelbehältnisse (A) sind vorzugsweise im Wesentlichen vollständig aus einem flexiblen Material gefertigt.

Bei den geeigneten Sammelbehältnissen (A) ist die Behältnisöffnung vorzugsweise in der Form eines Schlitzes ausgebildet, welcher zweckmäßigerweise im Wesentlichen parallel zum Behältnisboden und/oder zum verschlossenen oberen Behältnisende, insbesondere Behältnisrand, verläuft. Die Behältnisöffnung, insbesondere der Schlitz, kann dabei eine Ausstanzung oder einen Schnitt in der Behältniswandung darstellen. Vorzugsweise ist die Behältnisöffnung ausgelegt und eingerichtet, um metallische Einweg- und/oder Mehrweg-Instrumente, insbesondere metallische chirurgische Einweginstrumente, aufzunehmen und/oder um in dem Sammelbehältnis vorliegende metallische Einweg- und/oder Mehrweginstrumente, insbesondere metallische chirurgische Einweginstrumente, nicht durch Umstülpen wieder freizugeben. Vielfach hat es sich als vorteilhaft erwiesen, die Behältnisöffnung mit einem, insbesondere umlaufend, verstärkten Behältnisrand auszustatten.

Der Behältnisboden am unteren Behältnisende kann z.B. durch eine Faltung bzw. Bodenfalz gebildet werden. Alternativ kann der Behältnisboden durch eine Bodenwand darstellen. Bevorzugt ist der Behältnisboden auch verstärkt ausgebildet, beispielsweise durch einen doppel- oder mehrlagigen Abschnitt.

Die Haltevorrichtung kann in einer geeigneten Ausgestaltung auch ein Loch in dem Sammelbehältnis (A), insbesondere der Behältnisvorderwand und/oder der Behältnisrückwand, beispielsweise Griffloch, darstellen. Das Loch kann in einer Ausführungsform auch ausgelegt und eingerichtet sein, um eine Halteschlaufe oder -kordel hindurchzufädeln, welche dann zum temporären Befestigen des Sammelbehältnisses genutzt werden kann. Vorzugsweise wird das Loch in dem Sammelbehältnis derart ausgestaltet, dass ein Zugang zu dem Innenraum des Sammelbehältnisses über dieses Loch nicht möglich ist. Beispielsweise können die Lochränder von im Wesentlichen deckungsgleichen Löchern in Behältnisvorderwand und Behältnisrückwand umlaufend verschweißt oder verklebt sein.

Das Verbinden von Behältniswandungsabschnitten kann mittels Verklebens und/oder, insbesondere thermischen, Verschweißens und/oder Vernietens oder ähnlicher mechanischer Verbindungsmechanismen erfolgen.

Besonders bevorzugt sind geeignete Sammelbehältnisse (A) im Wesentlichen einstückig ausgebildet. Hierbei wird das Verschlusselement in der Regel außer Betracht gelassen. Das Verschlusselement für die Behältnisöffnung stellt vorzugsweise mindestens einen Klebestreifen dar.

Die Sammelbehältnisse (A) werden nach Befüllen mit benutzten und/oder kontaminierten metallischen Geräten aus Kliniken oder Arztpraxen vorzugsweise verschlossen. Die mindestens abschnittsweise, vorzugsweise im Wesentlichen vollständig, flexiblen, insbesondere verschließbaren, Sammelbehältnisse (A) können demgemäß für benutzte und/oder kontaminierte metallische medizinische Geräte, die in Kliniken anfallen, ebenso eingesetzt werden wie für entsprechend benutzte und/oder kontaminierte metallische medizinische Geräte aus Arztpraxen.

Insbesondere für den Einsatz im Klinikbetrieb hat es sich als vorteilhaft erwiesen, wenn die Sammelbehältnisse (A) eingerichtet und ausgelegt sind, um an einem Stationswagen temporär aufgehängt zu werden. Dies kann z.B. über integral in dem Sammelbehältnis vorliegende oder über extern angebrachte Halteschlaufen bewerkstelligt werden.

Vielfach hat es sich als besonders vorteilhaft erwiesen, die Öffnung der Sammelbehältnisse in der Form eines Schlitzes auszuführen, der in der Vorder- oder der Rückseitenwandung unterhalb des oberen Randes des Sammelbehältnisses vorliegt. Eine derartige Öffnung lässt sich durch einen Klebestreifen mit einem geeigneten Klebermaterial, das dem Fachmann bekannt ist, in einer Weise verschließen, dass ein Abtrennen des auf den die Öffnung aufgebrachten Klebestreifens nicht mehr oder nicht mehr zerstörungsfrei möglich ist. Eine schlitzförmige Öffnung im Sinne der vorliegenden Erfindung umfasst sowohl einen Schlitz als solchen, beispielsweise herbeigeführt durch einen Schnitt, wie auch eine Öffnung, die sich über die Breite des Sammelbehältnissen erstreckt und die gleichzeitig auch eine, insbesondere geringfügige, Erstreckung in Richtung von dem oberen Ende zu dem unteren Ende des Sammelbehältnisses aufweist. Derartige Öffnungen können beispielsweise mittels Ausstanzung erzeugt werden. Bevorzugt sind regelmäßig solche schlitzförmigen Öffnungen, die im Wesentlichen nicht über eine Erstreckung in Richtung von dem oberen Ende zu dem unteren Ende verfügen. Die sich gegenüberliegenden seitlichen Endpunkte der schlitzförmigen Öffnung können zum Beispiel in rundliche Ausnehmungen oder Ausstanzung münden. Hierdurch kann ein unbeabsichtigtes Einreißen der schlitzförmigen Öffnung unterbunden bzw. entgegengewirkt werden.

In einer bevorzugten Ausführungsform wird das Sammelbehältnis (A) nach der, insbesondere vollständigen, Befüllung und vorzugsweise spätestens vor der Überführung in das abschnittsweise oder vorzugsweise im Wesentlichen vollständig flexible Einweggebinde oder das starre Kunststoff- und/oder Metall-Einweg- oder -Mehrweggebinde (B), insbesondere - Mehrweggebinde, verschlossen, vorzugsweise irreversibel verschlossen.

Mit dem erfindungsgemäßen Verfahren ist es auch möglich, flexible Sammelbehältnisse (A) zu verwenden, die, insbesondere ausschließlich, nicht-infektiös kontaminierte metallische Abfälle enthalten. Bevorzugt sind hierbei Sammelbehältnisse (A), die, insbesondere ausschließlich, nicht-infektiös kontaminierte metallische Abfälle gemäß Abfallschlüssel AS 180104 nach Merkblatt Nr. 18 der Richtlinie über die ordnungsgemäße Entsorgung von Abfällen aus Einrichtungen des Gesundheitsdienstes gemäß Bundesgesundhbl. 2002, 51, 234 - 241, der Bund/Länder-Arbeitsgemeinschaft Abfall (LAGA) der Bundesrepublik Deutschland enthalten.

Vielfach hat es sich als zweckmäßig erwiesen, benutzte und/oder kontaminierte Kanülen und/oder Skalpelle nicht mit den vorangehend geschilderten flexiblen Sammelbehältnissen zu sammeln.

Die derart verschlossenen mindestens abschnittsweise, vorzugsweise im Wesentlichen vollständig, flexiblen Sammelbehältnisse (A) werden gemäß dem erfindungsgemäßen Verfahren in ein mindestens abschnittsweise, vorzugsweise im Wesentlichen vollständig, flexibles Einweggebinde oder ein starres Kunststoff- und/oder Metall-Einweg- oder -Mehrweggebinde (B), insbesondere -Mehrweggebinde, überführt. Dieses mindestens abschnittsweise, vorzugsweise im Wesentlichen vollständig, flexible Einweggebinde verfügt regelmäßig über Außenwände, die reißfest sind und die selbst bei beträchtlicher Krafteinwirkung keine Öffnungen, auch keine temporären Öffnungen bilden. Bei diesen flexiblen Einweggebinden handelt es sich um sogenannte Big Bags, auch bekannt unter flexible Bulk-Container handeln. Big Bags sind in der Regel mit einer quadratischen oder rechteckigen Grundfläche ausgestattet. Auch können sie über zwei oder mehr, beispielsweise vier Tragevorrichtungen, beispielsweise Schlaufen, verfügen. Diese sind zur besseren Kraftverteilung symmetrisch entlang des Umfangs des Öffnungsrandes verteilt und liegen beispielsweise an den Ecken eines Big Bags mit drei-, vier- oder mehreckiger Grundfläche vor. Darüber hinaus können auch Inlinerbeutel als flexible Einweggebinde (B) zum Einsatz kommen. Derartige Inlinerbeutel liegen vorzugsweise lösbar in einem starren Behältnis vor, insbesondere während des Sammelns, Lagerns und Transports der Sammelbehältnisse (A), beispielsweise auch in dem starren, insbesondere verschließbaren, Kunststoff- und/oder Metall-Einweg- oder -Mehrweggebinde (B). In einer zweckmäßigen Ausgestaltung sind diese mindestens abschnittsweise, vorzugsweise im Wesentlichen vollständig, flexiblen Einweggebinde mit einer Verschlussvorrichtung, beispielsweise mit einer Verschlussdecke, ausgestattet. Die abschnittsweise oder vorzugsweise im Wesentlichen vollständig flexiblen Einweggebinde und die starren Kunststoff- und/oder Metall-Einweg- oder -Mehrweggebinde (B), insbesondere -Mehrweggebinde, verfügen zweckmäßiger Weise über ein Aufnahmevolumen im Bereich von 50 bis 250 l, insbesondere im Bereich von 100 bis 200 l. Die flexiblen Einweggebinde (B) wie Big bag und Inlinerbeutel können in einer geeigneten Ausführungsform aus Bahnen bzw. Lagen aus gewebten Kunststofffasern oder -filamenten gebildet sein, insbesondere aus Polypropylenfasern oder -filamenten. Diese gewebten Bahnen bzw. Lagen können auch innen- und/oder außenseitig beschichtet sein, beispielsweise mit einer Metallschicht oder mit einer Lage aus hydrophobiertem Material. Bevorzugt wird diese Beschichtung innenseitig angebracht.

Sofern verschlossen, kann das mindestens abschnittsweise, vorzugsweise im Wesentlichen vollständig, flexible Einweggebinde oder das starre Kunststoff- und/oder Metall-Einweg- oder -Mehrweggebinde (B), insbesondere -Mehrweggebinde, zur Erzeugung eines größeren Maßes an Sicherheit auch verplombt werden.

Eine optionale, gleichwohl besonders zweckmäßige Behandlungsstufe des erfindungsgemäßen Verfahrens besteht darin, dass in Schritt f) jeweils ein einzelnes, insbesondere verschlossenes, mindestens abschnittsweise, vorzugsweise im Wesentlichen vollständig, flexibles Einweggebinde (B), enthaltend die mit benutzten und/oder kontaminierten metallischen medizinischen Geräten befüllten Sammelbehältnisse (A), oder eine Vielzahl an solchen, insbesondere verschlossenen, Einweggebinden (B) einem Pressschritt unter Ausbildung eines verpressten, insbesondere im Wesentlichen zylinderförmigen, Gebildes unterworfen wird. Auch kann der Inhalt der starren Kunststoff- und/oder Metall-Einweg- oder -Mehrweggebinde (B), insbesondere -Mehrweggebinde, in Form von mit benutzten und/oder kontaminierten metallischen medizinischen Geräten befüllten Sammelbehältnissen (A) einem solchen Pressschritt unterworfen werden. Für diesen Pressschritt kann auf herkömmliche Metallpressen zurückgegriffen werden. Die mit den gefüllten mindestens abschnittsweise, vorzugsweise im Wesentlichen vollständig, flexiblen Sammelbehältnissen (A) bestückten abschnittsweise oder vorzugsweise im Wesentlichen vollständig flexiblen Einweggebinde (B) bzw. diese gefüllten flexiblen Sammelbehältnisse (A) können in diesem Pressschritt in einer Weise zusammengepresst werden, dass allenfalls geringfügige Hohlräume in dem verpressten Gebilde verbleiben. Des Weiteren wird der Schritt des Verpressens vorzugsweise in einer Weise vorgenommen, dass das befüllte mindestens abschnittsweise, vorzugsweise im Wesentlichen vollständig, flexible Einweggebinde (B) hierbei nicht aufreißt oder aufplatzt.

Zwischen den Schritten d) und g) bzw. zwischen den Schritten e) bzw. f) und g) können zwecks Optimierung der Logistik Transport- und Lagerschritte vorgesehen sein. Demgemäß können in einer Ausführungsform eine Vielzahl der befüllten, insbesondere verschlossenen, abschnittsweise oder vorzugsweise im Wesentlichen vollständig flexiblen Einweggebinde (B) und/oder eine Vielzahl der befüllten starren, insbesondere verschlossenen, Kunststoff- und/oder Metall-Einweg- oder -Mehrweggebinde (B), insbesondere -Mehrweggebinde, gemäß d) bzw. e) oder f) zu einem ersten Lagerort, der ein Sammel- und/oder Zwischenlager darstellt, transportiert werden. Alternativ oder zusätzlich können eine Vielzahl der befüllten, insbesondere verschlossenen, abschnittsweise oder vorzugsweise im Wesentlichen vollständig flexiblen Einweggebinde (B) und/oder eine Vielzahl der befüllten starren, insbesondere verschlossenen, Kunststoff- und/oder Metall-Einweg- oder -Mehrweggebinde (B), insbesondere -Mehrweggebinde, gemäß d) bzw. e) oder f) und/oder die Vielzahl an dem ersten Lagerort gelagerter befüllter, insbesondere verschlossener, abschnittsweise oder vorzugsweise im Wesentlichen vollständig flexibler Einweggebinde (B) und/oder die Vielzahl der an dem ersten Lagerort gelagerten befüllten starren, insbesondere verschlossenen, Kunststoff- und/oder Metall-Einweg- oder -Mehrweggebinde (B), insbesondere -Mehrweggebinde, zu einem zweiten Lagerort, gegebenenfalls umfassend eine Metallpresse, transportiert werden. Optional können an diesem zweiten Lagerort die befüllten abschnittsweise oder vorzugsweise im Wesentlichen vollständig flexiblen Einweggebinde (B) und/oder der Inhalt der befüllten starren Kunststoff- und/oder Metall-Einweg- oder -Mehrweggebinde (B), insbesondere - Mehrweggebinde, in Aufnahmegebinde, die vorzugsweise größer als die abschnittsweise oder vorzugsweise im Wesentlichen vollständig flexiblen Einweggebinde (B) und/oder die Metall-Einweg- oder -Mehrweggebinde (B), insbesondere -Mehrweggebinde, sind, überführt werden, beispielsweise nach Verpressen der befüllten abschnittsweise oder vorzugsweise im Wesentlichen vollständig flexiblen Einweggebinde (B) und/oder des Inhalts der befüllten starren Kunststoff- und/oder Metall-Einweg- oder -Mehrweggebinde (B), insbesondere -Mehrweggebinde, in der Metallpresse. Ferner kann zusätzlich oder alternaitv die Vielzahl befüllter, insbesondere verschlossener, abschnittsweise oder vorzugsweise im Wesentlichen vollständig flexibler Einweggebinde (B) und/oder die Vielzahl der befüllten starren, insbesondere verschlossenen, Kunststoff- und/oder Metall-Einweg- oder -Mehrweggebinde (B), insbesondere -Mehrweggebinde, gemäß d) bzw. e) oder f) und/oder die Vielzahl an dem ersten Lagerort gelagerter befüllter, insbesondere verschlossener, abschnittsweise oder vorzugsweise im Wesentlichen vollständig flexibler Einweggebinde (B) und/oder die Vielzahl der an dem ersten Lagerort gelagerten befüllten starren, insbesondere verschlossenen, Kunststoff- und/oder Metall-Einweg- oder -Mehrweggebinde (B), insbesondere -Mehrweggebinde, und/oder die an dem zweiten Lagerort gelagerten befüllten, insbesondere verschlossenen, abschnittsweise oder vorzugsweise im Wesentlichen vollständig flexiblen Einweggebinde (B) und/oder die befüllten starren, insbesondere verschlossenen, Kunststoff- und/oder Metall-Einweg- oder -Mehrweggebinde (B), und/oder die am zweiten Lagerort gelagerten und mit den, gegebenenfalls verpressten, befüllten, insbesondere verschlossenen, abschnittsweise oder vorzugsweise im Wesentlichen vollständig flexiblen Einweggebinden (B), insbesondere - Mehrweggebinden, und/oder mit dem Inhalt der befüllten starren, insbesondere verschlossenen, Kunststoff- und/oder Metall-Einweg- oder -Mehrweggebinde (B), insbesondere - Mehrweggebinde, befüllten Aufnahmegebinde zu einem dritten Lagerort, umfassend eine Metallschmelze, transportiert werden. An diesem Lagerort können sodann die befüllten, gegebenenfalls verpressten, abschnittsweise oder vorzugsweise im Wesentlichen vollständig flexiblen Einweggebinde (B) und/oder der, gegebenenfalls verpresste, Inhalt der befüllten starren Kunststoff- und/oder Metall-Einweg- oder -Mehrweggebinde (B), insbesondere - Mehrweggebinde, und/oder der Inhalt der befüllten Aufnahmegebinde dort dem Verfahrensschritt g) unterworfen werden. Mit den Begriffen erster, zweiter und dritter Lagerort kann, muss aber nicht eine Transport- oder Lagerreihenfolge für die flexiblen oder starren Gebinde vorgegeben werden. Demgemäß können z.B. die Vielzahl der befüllten abschnittsweise oder vorzugsweise im Wesentlichen vollständig flexiblen Einweggebinde (B) oder eine Vielzahl der befüllten starren Kunststoff- und/oder Metall-Einweg- oder -Mehrweggebinde (B) gemäß d) bzw. e) oder f) untermittelbar an den dritten Lagerort verbracht und dort in der beschriebenen Weise dem Schmelzschritt unterworfen werden.

Beispielsweise kann sich eine (Zwischen)-Lagerung verpresster Gebilde anbieten, beispielsweise um abzuwarten, bis eine hinreichend große Anzahl an verpressten Gebilden vorliegt, die dem Folgeschritt der Metallschmelze zugeführt werden kann.

Auch kann es sich bei den ersten Lagerorten um eine Vielzahl an dezentralen Lagerorten handeln, von denen aus, beispielsweise sobald deren Kapazitätsgrenze erreicht ist, die Gebinde an den zweiten oder den dritten Lagerort verbracht werden. Bei dem zweiten Lagerort kann es sich z. B. um zertifizierte Lagerorte handeln, d.h. um solche, die Gebinde enthaltend benutzte und/oder kontaminierte medizinische Geräte handhaben, insbesondere umfüllen und/oder verpressen dürfen.

Die verpressten Gebilde können als solche dem Schmelzverfahren zugeführt werden. Hierbei hat sich als sehr vorteilhaft erwiesen, dass die benutzten und/oder kontaminierten metallischen medizinischen Geräte zunächst in dem mindestens abschnittsweise, vorzugsweise im Wesentlichen vollständig, flexiblen Sammelbehältnis (A) gesammelt und aufbewahrt werden. Diese Sammelbehältnisse, insbesondere wenn unter Verwendung der vorangehend spezifizierten Beutelmaterialien hergestellt, fallen ebenso wie die flexiblen Einweggebinde (B) in einer Menge an, dass die metallurgische Schmelze und die Rückgewinnung der Metalle - bzw. Einweggebinde hierdurch nicht beeinflusst oder beeinträchtigt werden. Das flexible Sammelbehältnismaterial wird in der Regel im metallurgischen Aufbereitungsprozess, d.h. dem Aufschmelzprozess der metallischen medizinischen Geräte, ohne toxische Rückstände zu bilden, verbrannt. Hierdurch können die Vorgaben gemäß LAGA M 18 - Vollzugshilfe zur Entsorgung von Abfällen aus Einrichtungen des Gesundheitsdienstes (Mitteilung der Bund/Länder-Arbeitsgemeinschaft Abfall; Stand Januar 2015; LAGA - Bund/Länder-Arbeitsgemeinschaft Abfall) und auch die Technischen Regeln für Biologische Arbeitsstoffe TRBA 250 betreffend Biologische Arbeitsstoffe im Gesundheitswesen und in der Wohlfahrtspflege der Bundesrepublik Deutschland erfüllt werden, welche das Entleeren und Sortieren von kontaminierten Klinikabfällen verbieten.

Mit der vorliegenden Erfindung geht die überraschende Erkenntnis einher, dass metallische medizinische Geräte wie Einweg- und auch Mehrweg-Instrumente einer Wiederverwertung zugeführt werden können, und zwar ohne dass diese Gegenstände zuvor auszusortieren sind. Durch Verwendung der mindestens abschnittsweise, vorzugsweise im Wesentlichen vollständig, flexiblen Sammelbehältnisse (A), insbesondere am Ort des Anfalls benutzter und/oder kontaminierter metallischer medizinischer Geräte, kombiniert mit dem Einsatz eines abschnittsweise oder vorzugsweise im Wesentlichen vollständig flexiblen Einweggebindes (B) oder eines starren Kunststoff- und/oder Metall-Einweg- oder -Mehrweggebindes (B), insbesondere -Mehrweggebindes, das bzw. dessen Inhalt vorzugsweise einem Pressschritt unterworfen wird, gelangt man nicht nur dahin, medizinische Einweg- und Mehrweginstrumente einer materialbezogenen Wiederverwertung zuzuführen. Vielmehr stellt das erfindungsgemäße Verfahren insbesondere auch eine sichere Handhabung benutzter und/oder kontaminierter medizinischer Geräte sowohl beim Klinikpersonal wie auch bei Mitarbeitern von Transport- und Recyclingunternehmen sicher. Mit der vorliegenden Erfindung geht insbesondere auch die überraschende Erkenntnis einher, dass in Schritt d) des erfindungsgemäßen Verfahrens nicht notwendiger Weise ein starres Metallgebinde einzusetzen ist, um einen verlässlichen und sicheren Transport zum Lagerort und zum Ort der Aufbereitung zu gewährleisten, sondern dass dieses bereits mit einem mindestens abschnittsweise, vorzugsweise im Wesentlichen vollständig, flexiblen Einweggebinde, beispielsweise einem sogenannten Big Bag, gelingt. Hierbei hat sich als besonders vorteilhaft erwiesen, dass man am Ort des Sammelns benutzter und/oder kontaminierter metallischer medizinischer Geräte mindestens abschnittsweise flexible Sammelbehältnisse (A) einsetzt, die derart gestaltet und vorzugsweise auch verschließbar sind, dass darin befindliche benutzte und/oder kontaminierte metallische medizinische Geräte sich nicht mehr entnehmen lassen und auch auf sonstige Weise nicht entweichen können. Ferner hat sich überraschend gezeigt, dass diese flexiblen Einweggebinde beim Aufschmelzen und Recycling der darin befindlichen metallischen Geräte nicht stören und hochreine Metalle auf ökonomische Art und Weise zurückgewonnen werden können. Mithilfe des erfindungsgemäßen Verfahrens wird ein sicherer und kostengünstiger Recyclingkreislauf zur Verfügung gestellt, mit dem es auf effiziente Art und Weise gelingt, insbesondere auch metallische medizinische Einweginstrumente einer weiteren Verwertung zuzuführen, sodass von der bisherigen finalen Entsorgung Abstand genommen werden kann.

Die in der vorstehenden Beschreibung und in den Ansprüchen offenbarten Merkmale der Erfindung können sowohl einzeln aus auch in jeder beliebigen Kombination für die Verwirklichung der Erfindung in ihren verschiedenen Ausführungsformen wesentlich sein.

## Patentansprüche

1. Verfahren zur recycelnden Entsorgung von metallischen medizinischen Geräten, umfassend die Schritte:
a1) Zurverfügungstellung mindestens eines mindestens abschnittsweise flexiblen Sammelbehältnisses (A) für benutzte oder kontaminierte metallische medizinische Geräte,
a2) Zurverfügungstellung mindestens eines abschnittsweise oder im Wesentlichen vollständig flexiblen Einweggebindes oder eines starren Kunststoff- und/oder Metall-Einweg- oder -Mehrweggebindes (B), eingerichtet und ausgelegt zur Aufnahme von mindestens zwei oder einer Vielzahl an mindestens abschnittsweise flexiblen Sammelbehältnissen (A), enthaltend benutzte oder kontaminierte metallische medizinische Geräte,
b) Sammeln von benutzten oder kontaminierten metallischen medizinischen Geräten in dem mindestens einen mindestens abschnittsweise flexiblen, Sammelbehältnis (A) oder in der Vielzahl an mindestens abschnittsweise flexiblen Sammelbehältnissen (A),
c) gegebenenfalls Verschließen des mit benutzten oder kontaminierten metallischen medizinischen Geräten befüllten mindestens einen mindestens abschnittsweise flexiblen Sammelbehältnisses (A) oder gegebenenfalls Verschließen der Vielzahl an mit benutzten oder kontaminierten metallischen medizinischen Geräten befüllten mindestens abschnittsweise flexiblen Sammelbehältnisse (A), **gekennzeichnet durch**
d) Überführen des mindestens einen mindestens abschnittsweise flexiblen Sammelbehältnisses (A) gemäß Schritt b) oder c) oder der Vielzahl an mindestens abschnittsweise flexiblen Sammelbehältnisse (A) gemäß Schritt b) oder c) in das abschnittsweise oder im Wesentlichen vollständig flexible Einweggebinde oder das starre Kunststoff- und/oder Metall-Einweg- oder -Mehrweggebinde (B),
wobei
in dem Fall, dass ein abschnittsweise oder im Wesentlichen vollständig flexibles Einweggebinde (B) zur Verfügung gestellt wird, dieses ein Big bag oder einen Inlinerbeutel zur Aufnahme der mindestens abschnittsweise flexiblen Sammelbehältnisse (A) umfasst oder darstellt, oder
in dem Fall, dass ein starres Metall-Einweg- oder -Mehrweggebindes (B) zur Verfügung gestellt wird, ein Inlinerbeutel in dem starren Metall-Einweg- oder - Mehrweggebinde (B) zur Aufnahme der mindestens abschnittsweise flexiblen Sammelbehältnisse (A) vorliegt,
e) gegebenenfalls Verschließen des mit dem mindestens einen Sammelbehältnis (A) oder mit der Vielzahl an mindestens abschnittsweise flexiblen Sammelbehältnissen (A) gemäß Schritt d) befüllten abschnittsweise oder im Wesentlichen vollständig flexiblen Einweggebindes oder des starren Kunststoff- und/oder Metall-Einweg- oder -Mehrweggebindes (B),
f) gegebenenfalls Unterwerfen des mindestens einen befüllten abschnittsweise oder im Wesentlichen vollständig flexiblen Einweggebindes (B) gemäß Schritt d) oder e) oder der in dem mindestens einen befüllten starren Kunststoff- und/oder Metall-Einweg- oder -Mehrweg-Gebinde (B) gesammelten, mit benutzten oder kontaminierten metallischen medizinischen Geräten befüllten mindestens abschnittsweise flexiblen Sammelbehältnisse (A) mindestens einem Pressschritt zur Volumenreduktion unter Erhalt eines verpressten Gebildes und
g) Aufschmelzen des Metalls des mindestens einen oder der Vielzahl an befüllten, gegebenenfalls verpressten, abschnittsweise oder im Wesentlichen vollständig flexiblen Einweggebinden (B) oder des Metalls der in dem mindestens einen oder der Vielzahl an befüllten starren Kunststoff- und/oder Metall-Einweg oder -Mehrweg-Gebinde (B) gesammelten, mit benutzten oder kontaminierten metallischen medizinischen Geräten befüllten, gegebenenfalls verpressten, mindestens abschnittsweise flexiblen Sammelbehältnisse (A) unter Rückgewinnung der aufgeschmolzenen Metalle.

2. Verfahren nach Anspruch 1, ferner umfassend
den Einsatz eines starren Aufnahmebehältnisses, insbesondere einer Metallgitterbox, für das abschnittsweise oder im Wesentlichen vollständig flexible Einweggebinde (B) sowie gegebenenfalls einer an der Unterseite des starren Aufnahmebehältnisses vorliegenden Tragevorrichtung, insbesondere Palette.

3. Verfahren nach Anspruch 1 oder 2, **dadurch gekennzeichnet, dass**
das mindestens abschnittsweise flexible Sammelbehältnis (A) im Wesentlichen feuchtigkeitsbeständig, dicht, insbesondere flüssigkeitsdicht, perforationsresistent, reißfest und/oder dauerhaft verschließbar ist.

4. Verfahren nach einem der vorangehenden Ansprüche, **dadurch gekennzeichnet, dass**
dass ein Inlinerbeutel in dem starren Kunststoff- Einweg- oder -Mehrweggebinde (B), zur Aufnahme der mindestens abschnittsweise flexiblen Sammelbehältnisse (A) vorliegt.

5. Verfahren nach einem der vorangehenden Ansprüche, **dadurch gekennzeichnet, dass**
in Schritt f) eine Vielzahl der abschnittsweise oder im Wesentlichen vollständig flexiblen befüllten Einweggebinde (B) dem Pressschritt unterworfen wird.

6. Verfahren nach einem der vorangehenden Ansprüche, **dadurch gekennzeichnet, dass**
eine Vielzahl der befüllten abschnittsweise oder im Wesentlichen vollständig flexiblen Einweggebinde (B) und/oder eine Vielzahl der befüllten starren Kunststoff- und/oder Metall-Einweg- oder -Mehrweggebinde (B) gemäß d) bzw. e) oder f) zu einem ersten Lagerort, der ein Sammel- und/oder Zwischenlager darstellt, transportiert werden; und/oder dass eine Vielzahl der befüllten abschnittsweise oder im Wesentlichen vollständig flexiblen Einweggebinde (B) und/oder eine Vielzahl der befüllten starren Kunststoff- und/oder Metall-Einweg- oder -Mehrweggebinde (B) gemäß d) bzw. e) oder f) und/oder die Vielzahl an dem ersten Lagerort gelagerter befüllter abschnittsweise oder im Wesentlichen vollständig flexibler Einweggebinde (B) und/oder die Vielzahl der an dem ersten Lagerort gelagerten befüllten starren Kunststoff- und/oder Metall-Einweg- oder - Mehrweggebinde (B) zu einem zweiten Lagerort, gegebenenfalls umfassend eine Metallpresse, transportiert werden; und/oder dass an diesem zweiten Lagerort die befüllten abschnittsweise oder im Wesentlichen vollständig flexiblen Einweggebinde (B) und/oder der Inhalt der befüllten starren Kunststoff- und/oder Metall-Einweg- oder -Mehrweggebinde (B) in Aufnahmegebinde, die vorzugsweise größer als die abschnittsweise oder im Wesentlichen vollständig flexiblen Einweggebinde (B) und/oder die Metall-Einweg- oder -Mehrweggebinde (B) sind, überführt werden, beispielsweise nach Verpressen der befüllten abschnittsweise oder im Wesentlichen vollständig flexiblen Einweggebinde (B) und/oder des Inhalts der befüllten starren Kunststoff- und/oder Metall-Einweg- oder -Mehrweggebinde (B) in der Metallpresse; und/oder dass die Vielzahl befüllter abschnittsweise oder im Wesentlichen vollständig flexibler Einweggebinde (B) und/oder die Vielzahl der befüllten starren Kunststoff- und/oder Metall-Einweg- oder -Mehrweggebinde (B) gemäß d) bzw. e) oder f) und/oder die Vielzahl an dem ersten Lagerort gelagerter befüllter abschnittsweise oder im Wesentlichen vollständig flexibler Einweggebinde (B) und/oder die Vielzahl der an dem ersten Lagerort gelagerten befüllten starren Kunststoff- und/oder Metall-Einweg- oder -Mehrweggebinde (B) und/oder die an dem zweiten Lagerort gelagerten befüllten abschnittsweise oder im Wesentlichen vollständig flexiblen Einweggebinde (B) und/oder die befüllten starren Kunststoff- und/oder Metall-Einweg- oder -Mehrweggebinde (B), und/oder die am zweiten Lagerort gelagerten und mit den, gegebenenfalls verpressten, befüllten abschnittsweise oder im Wesentlichen vollständig flexiblen Einweggebinden (B) und/oder mit dem Inhalt der befüllten starren Kunststoff- und/oder Metall-Einweg- oder -Mehrweggebinde (B) befüllten Aufnahmegebinde zu einem dritten Lagerort, umfassend eine Metallschmelze, transportiert werden; und/oder dass an diesem dritten Lagerort die befüllten, gegebenenfalls verpressten, abschnittsweise oder im Wesentlichen vollständig flexiblen Einweggebinde (B) und/oder der, gegebenenfalls verpresste, Inhalt der befüllten starren Kunststoff- und/oder Metall-Einweg- oder - Mehrweggebinde (B) und/oder der Inhalt der befüllten Aufnahmegebinde dem Verfahrensschritt g) unterworfen werden.

7. Verfahren nach einem der vorangehenden Ansprüche, **dadurch gekennzeichnet, dass**
die flexiblen Wandungsmaterialien des mindestens abschnittsweise flexiblen Sammelbehältnisses (A) ausgewählt sind aus der Gruppe bestehend aus Polyolefinlagen, insbesondere Polyethylenlagen; Polyesterlagen, insbesondere PET-Lagen; Polyolefin/Polyester-Lagen, insbesondere PE/PET-Lagen; Faservlieslagen, insbesondere umfassend oder gebildet aus Polyolefinfasern, insbesondere Polyethylenfasern, und/oder Polyesterfasern, insbesondere PET-Fasern, Laminatsystemen, enthaltend mindestens eine Papierlage, mindestens eine feuchtigkeitsbeständig beschichtete und/oder mit einer Metalllage beschichtete Papierlage und/oder mindestens eine Kunststofffolienlage, insbesondere enthaltend mindestens eine Papierlage und mindestens eine Kunststofffolienlage und/oder mindestens eine feuchtigkeitsbeständig beschichtete und/oder mit einer Metalllage beschichtete weitere Papierlage; ein- oder beidseitig in eine Kunststofflage eingebetteten Metallgeweben, Metallgewirken oder Metallvliesen; Lagen aus Polyvinylbutyrat, insbesondere recyceltem Polyvinylbutyrat, sowie deren beliebigen Mischungen.

8. Verfahren nach einem der vorangehenden Ansprüche, **dadurch gekennzeichnet, dass**
die metallischen medizinischen Geräte gebrauchte und/oder nicht verwendete Endoprothesen, chirurgische Instrumente, insbesondere Mehrweginstrumente, medizinische Einweginstrumente, Herzschrittmacher und/oder orthopädische Zubehörteile. metallische Einweg-Instrumente oder, insbesondere chirurgische, metallische Mehrweg-Instrumente darstellen oder umfassen.

9. Verfahren nach einem der vorangehenden Ansprüche, **dadurch gekennzeichnet, dass**
die mindestens abschnittsweise flexiblen Sammelbehältnisse (A) für benutzte und/oder kontaminierte metallische medizinische Geräte in Kliniken, medizinischen Ambulanzen und/oder Arztpraxen zur Verfügung gestellt und mit benutzten und/oder kontaminierten metallischen medizinischen Geräten befüllt werden.

10. Verfahren nach einem der vorangehenden Ansprüche, **dadurch gekennzeichnet, dass**
die mindestens abschnittsweise flexiblen Sammelbehältnisse (A) eingerichtet und ausgelegt sind, um benutzte und/oder kontaminierte metallische medizinische Geräte mit einem Gesamtgewicht im Bereich von 0,5 bis 5,0 kg, insbesondere im Bereich von 1,0 bis 3,0 kg, aufzunehmen, und/oder dass das abschnittsweise oder im Wesentlichen vollständig flexible Einweggebinde oder das starre Ein- oder Mehrweggebinde (B) ein Aufnahmevolumen im Bereich von 50 bis 250 l, insbesondere im Bereich von 100 bis 200 l, aufweist.

11. Verfahren nach einem der vorangehenden Ansprüche, **dadurch gekennzeichnet, dass**
die metallischen medizinischen Geräte Chrom und/oder Nickel enthalten, insbesondere mit Chrom und/oder Nickel legierte Edelstähle darstellen oder umfassen.

12. Verfahren nach einem der vorangehenden Ansprüche, **dadurch gekennzeichnet, dass**
die mindestens abschnittsweise flexiblen Sammelbehältnisse (A), insbesondere ausschließlich, nicht-infektiös kontaminierte metallische Abfälle, insbesondere gemäß Abfallschlüssel AS 180104 nach Merkblatt Nr. 18 der Richtlinie über die ordnungsgemäße Entsorgung von Abfällen aus Einrichtungen des Gesundheitsdienstes gemäß Bundesgesundhbl. 2002,51: 234-241, der Bund/Länder-Arbeitsgemeinschaft Abfall (LAGA) der Bundesrepublik Deutschland enthalten.

13. Verfahren nach einem der vorangehenden Ansprüche, **dadurch gekennzeichnet, dass**
das Sammelbehältnis (A) für metallische medizinische Geräte, insbesondere für metallische chirurgische Einweginstrumente, umfasst:
eine Behältnisvorderwand, eine Behältnisrückwand, gegebenenfalls eine erste Behältnisseitenwand, umfassend mindestens eine Seitenfalte, gegebenenfalls eine der ersten Behältnisseitenwand gegenüberliegende zweite Behältnisseitenwand, umfassend mindestens eine Seitenfalte,
ein unteres Behältnisende, umfassend einen Behältnisboden, und
einen dem Behältnisboden gegenüberliegendes verschlossenes oberes Behältnisende, wobei näher beabstandet zum verschlossenen oberen Behältnisende als zum Behältnisboden in einem Abschnitt der Behältnisvorderwand oder der Behältnisrückwand eine Behältnisöffnung vorliegt, die sich in Richtung von der ersten Behältnisseitenwand zu der zweiten Behältnisseitenwand erstreckt und wobei Behältnisvorderwand und Behältnisrückwand am unteren Behältnisende, insbesondere über eine Faltung, einstückig ineinander übergehen oder
wobei Behältnisvorderwand und Behältnisrückwand am unteren Behältnisende in einem sich von der ersten Behältnisseitenwand bis zur zweiten Behältnisseitenwand erstreckenden überlappenden ersten Abschnitt, insbesondere irreversibel, miteinander verbunden vorliegen und wobei der überlappende verbundene erste Abschnitt vollständig oder teilweise umgeschlagen auf und, insbesondere irreversibel, verbunden mit der Behältnisvorderwand oder die Behältnisrückwand vorliegt, und
wobei Behältnisvorderwand und Behältnisrückwand am verschlossenen oberen Behältnisende in einem sich von der ersten Behältnisseitenwand bis zur zweiten Behältnisseitenwand erstreckenden überlappenden zweiten Abschnitt zumindest teilweise, insbesondere irreversibel, miteinander verbunden vorliegen und wobei der überlappende zumindest teilweise verbundene zweite Abschnitt vollständig oder teilweise umgeschlagen auf und, insbesondere irreversibel, verbunden mit der Behältnisvorderwand oder die Behältnisrückwand vorliegt oder
wobei ein am oberen Behältnisende über die Behältnisrückwand hinausragender Abschnitt der Behältnisvorderwand unter Ausbildung des verschlossenen oberen Endes umgeschlagen auf und, insbesondere irreversibel, verbunden mit der Behältnisrückwand vorliegt oder wobei ein am oberen Behältnisende über die Behältnisvorderwand hinausragender Abschnitt der Behältnisrückwand unter Ausbildung des verschlossenen oberen Endes umgeschlagen auf und, insbesondere irreversibel, verbunden mit der Behältnisvorderwand vorliegt.

14. Verfahren nach Anspruch 13, **dadurch gekennzeichnet, dass**
das Sammelbehältnis (A) für metallische medizinische Geräte ferner mindestens ein Verschlusselement an oder auf der Behältniswand, welche die Behältnisöffnung enthält, diesseits der Behältnisöffnung in Bezug auf das obere Behältnisende, ausgelegt und eingerichtet, um die Behältnisöffnung, insbesondere irreversibel, zu verschließen, und mindestens eine Haltevorrichtung, insbesondere Griff, Schlaufe oder Aufnahmeöffnung oder -loch, ausgelegt und eingerichtet zum temporären Befestigen des mindestens abschnittsweise flexiblen Sammelbehältnisses (A), so dass bei gattungsgemäßem Gebrauch die Behältnisöffnung unterhalb des oberen Behältnisende vorliegt, umfasst.

15. Verfahren nach einem der vorangehenden Ansprüche, **dadurch gekennzeichnet, dass**
die starren Kunststoff- und/oder Metall-Mehrweggebinde (B) für das Sammeln und den Transport von mit benutzten und/oder kontaminierten metallischen medizinischen Geräten befüllten Sammelbehältnissen (A) wiederverwendet werden.

## Claims

1. A method for the recycling disposal of metallic medical devices, comprising the steps of:
a1) providing at least one at least sectionally flexible collection container (A) for used or contaminated metallic medical devices,
a2) providing at least one sectionally or substantially completely flexible disposable container or a rigid plastic and/or metal disposable or reusable container (B), arranged and designed for receiving at least two or a plurality of at least sectionally flexible collection containers (A) containing used or contaminated metallic medical devices,
b) collecting used or contaminated metallic medical devices in the at least one at least sectionally flexible collection container (A) or in the plurality of at least sectionally flexible collection containers (A),
c) optionally closing the at least one at least sectionally flexible collection container (A) filled with used or contaminated metallic medical devices or, if applicable, closing the plurality of at least sectionally flexible collection containers (A) filled with used or contaminated metallic medical devices, **characterized by**
d) transferring the at least one at least sectionally flexible collection container (A) according to step b) or c) or transferring the plurality of at least sectionally flexible collecting containers (A) according to step b) or c) into the sectionally or substantially completely flexible disposable container or into the rigid plastic and/or metal disposable or reusable container (B),
wherein
in the case where a sectionally or substantially completely flexible disposable container (B) is provided, the latter contains or represents a big bag or an inliner bag for receiving the at least sectionally flexible collection containers (A), or
in the case where a rigid metal disposable or reusable container (B) is provided, an inliner bag is placed in the rigid metal disposable or reusable container (B) for holding the at least sectionally flexible collection containers (A),
e) optionally, closing the sectionally or substantially completely flexible disposable container, which is filled with the at least one collection container (A) or with the plurality of at least sectionally flexible collection containers (A) according to step d), or the rigid plastic and/or metal disposable or reusable container (B),
f) optionally, subjecting the at least one filled sectionally or substantially completely flexible disposable container (B) according to step d) or e) or subjecting the at least sectionally flexible collection containers (A), which are filled with used or contaminated metallic medical devices and collected in the at least one filled rigid plastic and/or metal disposable or reusable container (B), to at least one pressing step for volume reduction while obtaining a compressed structure, and
g) melting of the metal of the at least one or the plurality of filled, optionally compressed, sectionally or substantially completely flexible disposable containers (B) or of the metal of the, optionally compressed, at least sectionally flexible collection containers (A), which are filled with used or contaminated metallic medical devices and collected in the at least one or the plurality of filled rigid plastic and/or metal disposable or reusable containers (B) under recovery of the melted metals.

2. Method according to claim 1, further comprising
the use of a rigid receiving container, in particular a metal mesh box, for the sectionally or substantially completely flexible disposable container (B), and, optionally, the use of a carrying device, in particular a pallet, present on the underside of the rigid receiving container.

3. Method according to claim 1 or 2, **characterized in that**
the at least sectionally flexible collection collection container (A) is essentially moisture-resistant, leakproof, in particular liquid-tight, resistant to perforation, tear-resistant and/or permanently closable.

4. Method according to one of the preceding claims, **characterized in that**
an inliner bag in the rigid plastic disposable or reusable container (B) is provided for receiving the at least sectionally flexible collecting containers (A).

5. Method according to one of the preceding claims, **characterized in that**
in step f), a plurality of the filled sectionally or substantially completely flexible disposable containers (B) is subjected to the pressing step.

6. Method according to one of the preceding claims, **characterized in that**
a plurality of the filled sectionally or substantially completely flexible disposable containers (B) and/or a plurality of the filled rigid plastic and/or metal disposable or reusable containers (B) according to d) or e) or f) are transported to a first storage location which is a collection and/or intermediate storage location; and/or **in that** a plurality of the filled sectionally or substantially completely flexible disposable containers (B) and/or a plurality of the filled rigid plastic and/or metal disposable or reusable containers (B) according to d) or e) or f) and/or the plurality of the filled sectionally or substantially completely flexible disposable containers (B) which are stored at the first storage location and/or the plurality of the filled rigid plastic and/or metal disposable or reusable containers (B) stored at the first storage location are transported to a second storage location which optionally comprises a metal press; and/or **in that**, at this second storage location, the filled sectionally or substantially completely flexible disposable containers (B) and/or the content of the filled rigid plastic and/or metal disposable or reusable containers (B) are transferred into receiving containers, which are preferably larger than the sectionally or substantially completely flexible disposable containers (B) and/or the metal disposable or reusable containers (B), for example after pressing the filled sectionally or substantially completely flexible disposable containers (B) and/or the contents of the filled rigid plastic and/or metal disposable or reusable containers (B) in the metal press; and/or **in that** the plurality of the filled sectionally or substantially completely flexible disposable containers (B) and/or the plurality of filled rigid plastic and/or metal disposable or reusable containers (B) according to d) or e) or f) and/or the plurality of the filled sectionally or substantially completely flexible disposable containers (B) which are stored at the first storage location and/or the plurality of the filled rigid plastic and/or metal disposable or reusable containers (B) which are stored at the first storage location and/or the filled sectionally or substantially completely flexible disposable containers (B) which are stored at the second storage location and/or the filled rigid plastic and/or metal disposable or reusable containers (B), and/or the receiving containers which are stored at the second storage location and are filled with the, optionally compressed, filled sectionally or substantially completely flexible disposable containers (B) and/or with the contents of the filled rigid plastic and/or metal disposable or reusable containers (B), are transported to a third storage location comprising a metal melt; and/or **in that** at this third storage location the filled, optionally compressed, sectionally or substantially completely flexible disposable containers (B) and/or the, optionally compressed, content of the filled rigid plastic and/or metal disposable or reusable containers (B) and/or the content of the filled receiving containers are subjected to process step g).

7. Method according to one of the preceding claims, **characterized in that**
the flexible wall materials of the at least sectionally flexible collecting container (A) are selected from the group consisting of polyolefin layers, in particular polyethylene layers; polyester layers, in particular PET layers; polyolefin/polyester layers, in particular PE/PET layers; nonwoven fiber layers, in particular comprising or formed from polyolefin fibers, in particular polyethylene fibers, and/or polyester fibers, in particular PET fibers, laminate systems containing at least one paper layer, at least one paper layer coated to be moisture-resistant and/or coated with a metal layer and/or at least one plastic film layer, in particular containing at least one paper layer and at least one plastic film layer and/or at least one further paper layer coated to be moisture-resistant and/or coated with a metal layer; metal fabrics, knitted metal fabrics or nonwoven metal fabrics embedded on one or both sides in a plastic layer; layers of polyvinyl butyrate, in particular recycled polyvinyl butyrate, and any mixtures thereof.

8. Method according to one of the preceding claims, **characterized in that**
the metallic medical devices are or comprise used and/or unused endoprostheses, surgical instruments, in particular reusable instruments, disposable medical instruments, cardiac pacemakers and/or orthopaedic accessories, metallic disposable instruments or, in particular surgical, metallic reusable instruments.

9. Method according to one of the preceding claims, **characterized in that**
the at least sectionally flexible collection containers (A) for used and/or contaminated metallic medical devices are made available in clinics, medical outpatient departments and/or medical practices and are filled with used and/or contaminated metallic medical devices.

10. Method according to one of the preceding claims, **characterized in that**
the at least sectionally flexible collection containers (A) are arranged and designed to hold used and/or contaminated metallic medical devices with a total weight in the range from 0.5 to 5.0 kg, in particular in the range from 1.0 to 3.0 kg, and/or **in that** the at least sectionally or substantially completely flexible disposable container or the rigid disposable or reusable container (B) has a holding volume in the range from 50 to 250 1, in particular in the range from 100 to 200 1.

11. Method according to one of the preceding claims, **characterized in that**
the metallic medical devices contain chromium and/or nickel, in particular are or comprise stainless steels alloyed with chromium and/or nickel.

12. Method according to one of the preceding claims, **characterized in that**
the at least sectionally flexible collection containers (A) contain, in particular exclusively, non-infectiously contaminated metallic waste, in particular according to waste code AS 180104 according to information sheet No. 18 of the directive on the proper disposal of waste from health service facilities according to Bundesgesundhbl. 2002, 51: 234-241, of the Bund/Länder-Arbeitsgemeinschaft Abfall (LAGA) of the Federal Republic of Germany.

13. Method according to one of the preceding claims, **characterized in that** the collecting container (A) for metallic medical devices, in particular for metal disposable surgical instruments, comprises:
a container front wall, a container rear wall, optionally a first container side wall which comprises at least one side fold, optionally a second container side wall opposite the first container side wall which comprises at least one side fold, a lower container end which comprises a container base, and a closed upper container end opposite the container base, wherein a container opening is present in a section of the container front wall or the container rear wall at a closer distance from the closed upper container end than from the container base, which container opening extends in the direction from the first container side wall to the second container side wall, and wherein the container front wall and the container rear wall merge integrally into one another at the container lower end, in particular via a fold, or wherein the container front wall and the container rear wall are , in particular irreversibly, connected to one another at the container lower end in an overlapping first section extending from the container first side wall to the container second side wall and wherein the overlapping connected first section is present completely or partially folded over onto and, in particular irreversibly, connected to the container front wall or the container rear wall, and
wherein the container front wall and the container rear wall are at least partially, in particular irreversibly, connected to one another at the closed upper container end in an overlapping second section extending from the first container side wall to the second container side wall, and wherein the overlapping at least partially connected second section is present completely or partially folded over onto and, in particular irreversibly, connected to the container front wall or the container rear wall, or wherein a section of the container front wall projecting beyond the container rear wall at the upper end of the container is present folded over onto and, in particular irreversibly, connected to the container rear wall thereby forming the closed upper end, or wherein a section of the container rear wall projecting beyond the container front wall at the upper end of the container is present folded over onto and, in particular irreversibly, connected to the container front wall thereby forming the closed upper end.

14. Method according to claim 13, **characterized in that**
the collection container (A) for metallic medical devices further comprises at least one closure element at or on the container wall comprising the container opening, on the side of the container opening with respect to the upper container end, designed and arranged to close the container opening, in particular irreversibly, and at least one holding device, in particular a handle, loop or receiving opening or hole, designed and arranged to temporarily fasten the at least sectionally flexible collection container (A), so that the container opening is positioned below the upper end of the container when used as intended.

15. Method according to one of the preceding claims, **characterized in that**
the rigid plastic and/or metal reusable containers (B) are reused for the collection and transport of collection containers (A) filled with used and/or contaminated metallic medical devices.

## Revendications

1. Procédé de dépollution par valorisation d'appareils médicaux métalliques, comprenant les étapes suivantes :
a1) la mise à disposition d'au moins un récipient collecteur (A) au moins partiellement souple pour appareils médicaux métalliques usés et/ou contaminés,
a2) la mise à disposition d'au moins un récipient jetable partiellement ou essentiellement entièrement souple ou d'un récipient rigide jetable ou réutilisable (B) en plastique et/ou en métal, disposé et conçu pour recevoir au moins deux ou une pluralité de récipients collecteurs (A) au moins partiellement souples, contenant des appareils médicaux métalliques usés et/ou contaminés,
b) la collecte des appareils médicaux métalliques usés et/ou contaminés dans l'au moins un récipient collecteur (A) au moins partiellement souple ou dans la pluralité de récipients collecteurs (A) au moins partiellement souples,
c) la fermeture, le cas échéant, de l'au moins un récipient collecteur (A) au moins partiellement souple rempli d'appareils médicaux métalliques usés et/ou contaminés, ou la fermeture, le cas échéant, de la pluralité de récipients collecteurs (A) au moins partiellement souples remplis d'appareils médicaux métalliques usés et/ou contaminés, **caractérisé par**
d) le transfert de l'au moins un récipient collecteur (A) au moins partiellement souple selon l'étape b) ou c) ou de la pluralité de récipients collecteurs (A) au moins partiellement souples selon l'étape b) ou c) dans le récipient jetable partiellement ou essentiellement entièrement souple ou le récipient rigide jetable ou réutilisable (B) en plastique et/ou en métal, dans lequel
dans le cas où un récipient jetable (B) partiellement ou essentiellement entièrement souple a été mis à disposition, celui-ci comprend ou est un Big bag ou un sac intérieur pour accueillir les récipients collecteurs (A) au moins partiellement souples, ou
dans le cas où un récipient rigide jetable ou réutilisable (B) en métal a été mis à disposition, un sac intérieur est prévu dans le récipient rigide jetable ou réutilisable (B) en métal pour accueillir les récipients collecteurs (A) au moins partiellement souples,
e) la fermeture, le cas échéant, du récipient jetable partiellement ou essentiellement entièrement souple rempli avec l'au moins un récipient collecteur (A) ou avec la pluralité de récipients collecteurs (A) au moins partiellement souples selon l'étape d), ou du récipient rigide jetable ou réutilisable (B) en plastique et/ou en métal,
f) la soumission, le cas échéant, de l'au moins un récipient jetable (B) partiellement ou essentiellement entièrement souple rempli selon l'étape d) ou e), ou des récipients collecteurs (A) au moins partiellement souples remplis d'appareils médicaux métalliques usés et/ou contaminés collectionnés dans l'au moins un récipient rigide jetable ou réutilisable (B) en plastique et/ou en métal rempli, à au moins une étape de compression destinée à la réduction du volume pour obtenir une structure comprimée, et
g) la fusion du métal de l'au moins un ou de la pluralité de récipients jetables (B) partiellement ou essentiellement entièrement souples remplis, éventuellement pressés, ou du métal des récipients collecteurs (A) au moins partiellement souples remplis d'appareils médicaux métalliques usés et/ou contaminés collectionnés, éventuellement pressés, dans l'au moins un ou dans la pluralité de récipients rigides jetables ou réutilisables (B) en plastique et/ou en métal remplis, en récupérant les métaux fondus.

2. Procédé selon la revendication 1, comprenant en outre l'utilisation d'un récipient rigide, notamment une boîte en treillis métallique, pour le récipient jetable (B) partiellement ou essentiellement entièrement souple, ainsi que, le cas échéant, un dispositif de support, notamment une palette, prévu à la face inférieure du récipient rigide.

3. Procédé selon la revendication 1 ou 2, **caractérisé en ce que**
le récipient collecteur (A) au moins partiellement souple est essentiellement résistant à l'humidité, étanche, notamment étanche aux liquides, résistant aux perforations, résistant aux déchirures, et/ou apte à être fermé de manière permanente.

4. Procédé selon l'une quelconque des revendications précédentes, **caractérisé en ce que**
un sac intérieur est prévu dans le récipient rigide jetable ou réutilisable (B) en plastique, pour accueillir les récipients collecteurs (A) au moins partiellement souples.

5. Procédé selon l'une quelconque des revendications précédentes, **caractérisé en ce que**
dans l'étape f), une pluralité des récipients jetables (B) partiellement ou essentiellement entièrement souples remplis sont soumis à l'étape de compression.

6. Procédé selon l'une quelconque des revendications précédentes, **caractérisé en ce que**
une pluralité des récipients jetables (B) partiellement ou essentiellement entièrement souples remplis et/ou une pluralité des récipients rigides jetables ou réutilisables (B) en plastique et/ou en métal remplis selon d) resp. e) ou f) sont transportés vers un premier lieu de stockage, qui est une installation de collecte et/ou de stockage provisoire ; et/ou **en ce qu'**une pluralité des récipients jetables (B) partiellement ou essentiellement entièrement souples remplis et/ou une pluralité des récipients rigides jetables ou réutilisables (B) en plastique et/ou en métal remplis selon d) resp. e) ou f), et/ou la pluralité des récipients jetables (B) partiellement ou essentiellement entièrement souples remplis stockés dans le premier lieu de stockage et/ou la pluralité des récipients rigides jetables ou réutilisables (B) en plastique et/ou en métal remplis stockés dans le premier lieu de stockage, sont transportés vers un deuxième lieu de stockage, comprenant éventuellement une presse à métaux ; et/ou **en ce que** à ce deuxième lieu de stockage, les récipients jetables (B) partiellement ou essentiellement entièrement souples remplis et/ou le contenu des récipients rigides jetables ou réutilisables (B) en plastique et/ou en métal remplis sont transférés dans des récipients de réception, qui sont de préférence plus grands que les récipients jetables (B) partiellement ou essentiellement entièrement souples et/ou les récipients rigides jetables ou réutilisables (B) en métal, par exemple après avoir pressé les récipients jetables (B) partiellement ou essentiellement entièrement souples remplis et/ou le contenu des récipients rigides jetables ou réutilisables (B) en plastique et/ou en métal remplis dans la presse à métaux ; et/ou **en ce que** la pluralité des récipients jetables (B) partiellement ou essentiellement entièrement souples remplis et/ou la pluralité des récipients rigides jetables ou réutilisables (B) en plastique et/ou en métal remplis selon d) resp. e) ou f), et/ou la pluralité des récipients jetables (B) partiellement ou essentiellement entièrement souples remplis stockés dans le premier lieu de stockage, et/ou la pluralité des récipients rigides jetables ou réutilisables (B) en plastique et/ou en métal remplis stockés dans le premier lieu de stockage, et/ou les récipients jetables (B) partiellement ou essentiellement entièrement souples remplis stockés dans le deuxième lieu de stockage et/ou les récipients rigides jetables ou réutilisables (B) en plastique et/ou en métal remplis, et/ou les récipients de réception stockés dans le deuxième lieu de stockage et remplis des récipients jetables (B) partiellement ou essentiellement entièrement souples remplis et éventuellement pressés, et/ou remplis du contenu des récipients rigides jetables ou réutilisables (B) en plastique et/ou en métal remplis, sont transportés vers un troisième lieu de stockage, comprenant un métal fondu ; et/ou **en ce que** à ce troisième lieu de stockage, les récipients jetables (B) partiellement ou essentiellement entièrement souples remplis, éventuellement pressés, et/ou le contenu éventuellement pressé des récipients rigides jetables ou réutilisables (B) en plastique et/ou en métal remplis, et/ou le contenu des récipients de réception remplis est/sont soumis à l'étape de procédé g).

7. Procédé selon l'une quelconque des revendications précédentes, **caractérisé en ce que**
les matériaux de paroi souples du récipient collecteur (A) au moins partiellement souple sont choisis dans le groupe constitué de couches de polyoléfine, en particulier de couches de polyéthylène ; de couches de polyester, en particulier de couches de PET ; de couches de polyoléfine/polyester, en particulier de couches de PE/PET ; de couches de fibres non tissées, en particulier comprenant ou formées de fibres de polyoléfine, en particulier de fibres de polyéthylène, et/ou de fibres de polyester, en particulier de fibres de PET, de systèmes stratifiés, comprenant au moins une couche de papier, au moins une couche de papier couché résistant à l' humidité et/ou recouvert d'une couche métallique et/ou au moins une couche de film plastique, en particulier comprenant au moins une couche de papier et au moins une couche de film plastique et/ou au moins une couche additionnelle de papier couché résistant à l' humidité et/ou recouvert d'une couche métallique ; de tissus métalliques, tricots métalliques ou non-tissés métalliques noyés sur une ou deux faces dans une couche de matière plastique ; de couches en polyvinylbutyrate, en particulier en polyvinylbutyrate recyclé, et tous mélanges de ceux-ci.

8. Procédé selon l'une quelconque des revendications précédentes, **caractérisé en ce que**
les appareils médicaux métalliques sont ou comprennent des endoprothèses usagées et/ou non utilisées, des instruments chirurgicaux, en particulier des instruments réutilisables, des instruments médicaux à usage unique, des stimulateurs cardiaques et/ou des accessoires orthopédiques, des instruments métalliques à usage unique ou, en particulier, des instruments chirurgicaux métalliques réutilisables.

9. Procédé selon l'une quelconque des revendications précédentes, **caractérisé en ce que**
les récipients collecteurs (A) au moins partiellement souples pour appareils médicaux métalliques usés et/ou contaminés sont mis à disposition et replis d'appareils médicaux métalliques usés et/ou contaminés dans des cliniques, des cliniques externes et/ou des cabinets médicaux.

10. Procédé selon l'une quelconque des revendications précédentes, **caractérisé en ce que**
les récipients collecteurs (A) au moins partiellement souples sont disposés et conçus pour recevoir des appareils médicaux métalliques usés et/ou contaminés d'un poids total compris dans la plage de 0,5 bis 5,0 kg, notamment dans la plage de 1,0 bis 3,0 kg, et/ou **en ce que** le récipient jetable partiellement ou essentiellement entièrement souple ou le récipient rigide jetable ou réutilisable (B) a un volume de réception dans la plage de 50 bis 250 l, notamment dans la plage de 100 bis 200 l.

11. Procédé selon l'une quelconque des revendications précédentes, **caractérisé en ce que**
les appareils médicaux métalliques consistent ou comprennent du chrome et/ou du nickel, en particulier des aciers inoxydables alliés au chrome et/ou au nickel.

12. Procédé selon l'une quelconque des revendications précédentes, **caractérisé en ce que**
les récipients collecteurs (A) au moins partiellement souples contiennent, en particulier exclusivement, des déchets métalliques contaminés non infectieux, en particulier conformément au Code de déchets AS 180104 selon la feuille d'instruction n° 18 de la directive sur l'élimination appropriée des déchets des établissements de santé, conformément au journal du ministère fédéral de la santé 2002,51 : 234-241 de la Bund/Länder-Arbeitsgemeinschaft Abfall (LAGA) de la République fédérale d'Allemagne.

13. Procédé selon l'une quelconque des revendications précédentes, **caractérisé en ce que**
le récipient collecteur (A) pour appareils médicaux métalliques, notamment pour appareils médicaux métalliques à usage unique, comprend :
une paroi avant du récipient, une paroi arrière du récipient, éventuellement une première paroi latérale du récipient, comprenant au moins un pli latéral, éventuellement une deuxième paroi latérale du récipient opposée à la première paroi latérale du récipient, comprenant au moins un pli latéral,
une extrémité inférieure du récipient, comprenant un fond de récipient, et
une extrémité supérieure du récipient fermée, opposée au fond de récipient,
dans lequel, plus près de l'extrémité supérieure du récipient fermée que du fond de récipient, dans une section de la paroi avant du récipient ou de la paroi arrière du récipient, se trouve une ouverture de récipient, qui s'étend dans la direction de la première paroi latérale du récipient vers la deuxième paroi latérale du récipient, et
dans lequel la paroi avant du récipient et la paroi arrière du récipient se confondent mutuellement de manière à former une seule pièce à l'extrémité inférieure du récipient, notamment par un pli, ou
dans lequel la paroi avant du récipient et la paroi arrière du récipient sont raccordées l'une à l'autre, notamment de manière irréversible, à l'extrémité inférieure du récipient, dans une première section se chevauchant s'étendant de la première paroi latérale du récipient à la deuxième paroi latérale du récipient, et
dans lequel la première section se chevauchant raccordée est totalement ou partiellement rabattue sur, et raccordée à, la paroi avant du récipient ou à la paroi arrière du récipient, notamment de manière irréversible, et
dans lequel la paroi avant du récipient et la paroi arrière du récipient sont au moins partiellement raccordées l'une à l'autre, notamment de manière irréversible, à l'extrémité supérieure du récipient fermée, dans une deuxième section se chevauchant s'étendant de la première paroi latérale du récipient à la deuxième paroi latérale du récipient, et
dans lequel la deuxième section se chevauchant, au moins partiellement raccordée, est totalement ou partiellement rabattue sur, et raccordée à, la paroi avant du récipient ou à la paroi arrière du récipient, notamment de manière irréversible, ou
dans lequel une section de la paroi avant du récipient faisant saillie à l'extrémité supérieure du récipient sur la paroi arrière du récipient est rabattue sur, et raccordée à, la paroi arrière du récipient, notamment de manière irréversible, formant l'extrémité supérieure fermée, ou
dans lequel une section de la paroi arrière du récipient faisant saillie à l'extrémité supérieure du récipient sur la paroi avant du récipient est rabattue sur, et raccordée à, la paroi avant du récipient, notamment de manière irréversible, formant l'extrémité supérieure fermée.

14. Procédé selon la revendication 13, **caractérisé en ce que**
le récipient collecteur (A) pour appareils médicaux métalliques comprend en outre au moins un élément de fermeture à ou sur la paroi de récipient dans laquelle se trouve l'ouverture de récipient, du même côté de l'ouverture de récipient que l'extrémité supérieure du récipient, conçu et aménagé pour fermer l'ouverture de récipient, notamment de manière irréversible, et au moins un dispositif de maintien, notamment une poignée, une boucle ou une ouverture ou un trou de réception, conçu et aménagé pour fixer temporairement le récipient collecteur (A) au moins partiellement souple, de sorte que, lorsque utilisé comme prévu, l'ouverture de récipient se trouve en dessous de l'extrémité supérieure du récipient.

15. Procédé selon l'une quelconque des revendications précédentes, **caractérisé en ce que**
les récipients rigides réutilisables (B) en plastique et/ou en métal sont réutilisés pour la collecte et le transport de récipients collecteurs (A) remplis d'appareils médicaux métalliques usés et/ou contaminés.
